Europäisches Patentamt

European Patent Office

Office européen des brevets

(19)

(11) Numéro de publication: **0 097 748**

**A1**

## (12) DEMANDE DE BREVET EUROPEEN

(21) Numéro de dépôt: 82401196.9

(22) Date de dépôt: 28.06.82

(51) Int. Cl.³: **A 61 B 5/14**
**A 61 B 17/32**

(43) Date de publication de la demande:
11.01.84 Bulletin 84/2

(84) Etats contractants désignés:
AT BE CH DE GB IT LI LU NL SE

(71) Demandeur: Slama, Gérard Joseph
17 avenue du Château
F-94210 La Varenne Saint Hilaire (Val de Marne)(FR)

(72) Inventeur: Slama, Gérard Joseph
17 avenue du Château
F-94210 La Varenne Saint Hilaire (Val de Marne)(FR)

(74) Mandataire: Rataboul, Michel
Cabinet Michel Rataboul 69, rue de Richelieu
F-75002 Paris(FR)

(54) Dispositif pour provoquer une petite piqure en vue de recueillir une goutte de sang.

(57) Pour effectuer une petite piqûre en vue de recueillir une goutte de sang, on arme une monture mobile (3) portant une aiguille (1) et sollicitée par un ressort.

Dans la position de repos du ressort représenté au dessin, l'aiguille (1) est en deça du bord (8) de l'extrémité (9) et il subsiste une distance $x$ entre une tige radiale (5) (portant un bouton de commande 5a) et une butée 7.

Lorsque l'on déclenche le mécanisme, le ressort pousse la monture (3) brusquement et la tige (5) dépasse la position de repos pour atteindre la butée (7) qui limite sa course, de sorte que l'aiguille (1) sort, atteint une position extrême déterminée et revient automatiquement à sa position abritée.

FIG.2

# DISPOSITIF POUR PROVOQUER UNE PETITE PIQURE
## EN VUE DE RECUEILLIR UNE GOUTTE DE SANG

Les patients atteints de certaines maladies, et tout particulièrement du diabète, doivent plusieurs fois par jour vérifier la teneur en sucre de leur sang.

Selon cette teneur, le patient doit s'administrer certains médicaments ou pas.

Etant donné la fréquence de ces vérifications, il est indispensable que le patient ait sur lui un dispositif portatif, d'utilisation facile et de fonctionnement sûr car il n'est pas imaginable qu'il doive faire appel à un tiers et moins encore à un laboratoire spécialisé pour procéder à ces vérifications.

C'est pourquoi, il existe déjà de tels dispositifs portatifs mais ils ne donnent pas entière satisfaction tout particulièrement en ce qui concerne leur encombrement relatif et leur facilité d'emploi.

On connaît ainsi, par exemple, un dispositif qui comprend un corps et une monture mobile porte-pointe qui pivote par rapport au corps selon un mouvement qui rappelle celui des chiens de fusil et qui est sollicité par un ressort vers sa position active.

La monture est extérieure par rapport au corps et, comme elle porte une pointe, cette dernière peut accidentellement blesser l'usager même lorsqu'elle est immobile c'est-à-dire indépendamment de toute action involontaire sur un organe de commande qu'il faut manoeuvrer pour libérer la monture au moment voulu pour que la pointe s'enfonce très légèrement dans la peau.

On connaît également un autre dispositif dont la monture est intérieure au corps mais celui-ci est obtenu par assemblage de demi-coquilles en matière synthétique rigide de forme parallélépipédique et est relativement encombrant.

En outre, sa forme et ses dimensions le rendent peu apte à un nettoyage complet.

On connaît aussi le brevet allemand 459.483 qui décrit un dispositif du même type que celui de la présente invention, mais la butée m qui limite l'extraction de la pointe d hors du corps a, est constituée par un écrou plus ou moins vissé sur une tige filetée b de sorte que cette butée, loin de constituer un point fixe indéréglable, est soumise lors de chaque utilisation à des chocs contre la partie supérieure e du corps a en risquant ainsi un déplacement involontaire rendant aléatoire la sortie de la pointe d.

Avec une telle structure, il n'existe aucun point de repère sûr, auquel l'utilisateur peut se fier.

Ainsi, l'utilisateur est obligé d'effectuer un réglage plus ou moins empirique (et de toute façon incertain) chaque fois qu'il doit utiliser le dispositif.

Une autre différence fondamentale vient du fait qu'une partie importante du dispositif est située à l'extérieur du corps a, c'est-à-dire: la tige filetée b sur toute sa longueur, l'écrou m et un bouchon non référencé situé à l'extrémité de la tige filetée b.

Cette disposition encombrante et peu commode vient de la conception même du dispositif, car dès lors qu'il s'agit de régler l'extraction de la pointe d au moyen de l'écrou m, il est impossible de prévoir ce dernier à l'intérieur du corps a puisqu'alors il serait inaccessible et invisible.

La présente invention remédie à ces inconvénients, du fait que l'extraction de la pointe est limitée de manière indéréglable par une butée fixe qui détermine l'extraction maximum possible.

L'invention a pour objet un dispositif pour provoquer une petite piqûre au moyen d'une pointe en vue de recueillir une goutte de sang devant être étudiée, notamment par mise en contact avec un réactif, du type comprenant un corps dans lequel une monture porte-pointe est montée mobile et est sollicitée par un ressort entre une position d'effacement à l'intérieur du corps et une position d'extraction maximum hors du corps déterminée par une butée avec laquelle la monture ou une pièce solidaire de cette dernière, doit coopérer pour déterminer la position d'extraction maximum, au-delà de la position naturelle déterminée par la longueur du ressort au repos, caractérisé en ce que la butée est fixe tandis que la longueur totale du corps est réglable au moyen d'une pièce reliée au corps, ajustable en position mais indépendante des pièces mobiles.

Selon d'autres caractéristiques de l'invention:

- la pièce est constituée par un manchon situé à l'extrémité du corps où se trouve l'orifice par lequel la pointe doit s'extraire;

- la pièce porte une graduation située en regard d'un repère du corps;

- le corps a la forme générale d'un stylographe et il comprend un capuchon fermé par une cloison transversale pour constituer un réceptacle susceptible d'être obturé par un bouchon et devant contenir des bandelettes de tout type connu en soi et destinées à recevoir par capillarité la goutte de sang apparaissant après piqûre, ce réceptacle pouvant être muni intérieurement d'un produit absorbant l'humidité tel qu'une capsule dessiccante.

L'invention sera mieux comprise par la description détaillée ci-après faite en référence au dessin annexé. Bien entendu, la description et le dessin ne sont donnés qu'à titre d'exemple indicatif et non limitatif.

- la figure 1 est une vue schématique en coupe du corps d'un dispositif conforme à l'invention représenté dans la situation qui correspond à la position stable de repos du ressort;

- la figure 2 est une vue schématique extérieure avec coupe partielle du même corps dans la même situation du ressort;

- la figure 3 est une vue schématique avec coupe partielle montrant le même corps, le ressort étant dans sa position de tension maximum, c'est-à-dire lorsque la monture est en position armée;

- la figure 4 est une vue schématique avec coupe partielle montrant le dispositif en cours d'utilisation, pendant le bref instant de la brusque détente du ressort qui provoque la sortie de la pointe au-delà de l'extrémité du corps;

- la figure 5 est une vue schématique avec coupe partielle montrant l'association du corps du dispositif avec un capuchon contenant une réserve de bandelettes.

En se reportant au dessin, on voit qu'un dispositif conforme à l'invention comprend une pointe 1 solidaire d'une tige 1a par laquelle elle peut être assujettie d'une manière amovible à une monture mobile porte-pointe 3 qui est constituée, ici, par un cylindre monté coulissant dans l'intérieur lui-même cylindrique d'un corps 2 en forme de stylographe.

Un ressort 4 est placé dans le corps 2 et prend appui d'une part contre le fond de ce corps 2 et d'autre part sur la monture 3, laquelle est munie d'une tige radiale 5 terminée par un bouton 5a et qui constitue un organe de commande.

La tige 5 est engagée dans une lumière 2a du corps 2 dont la forme, bien connue en soi, est telle qu'elle présente une première butée d'arrêt 6 et une seconde butée d'arrêt 7.

Le fonctionnement de ce dispositif est le suivant:

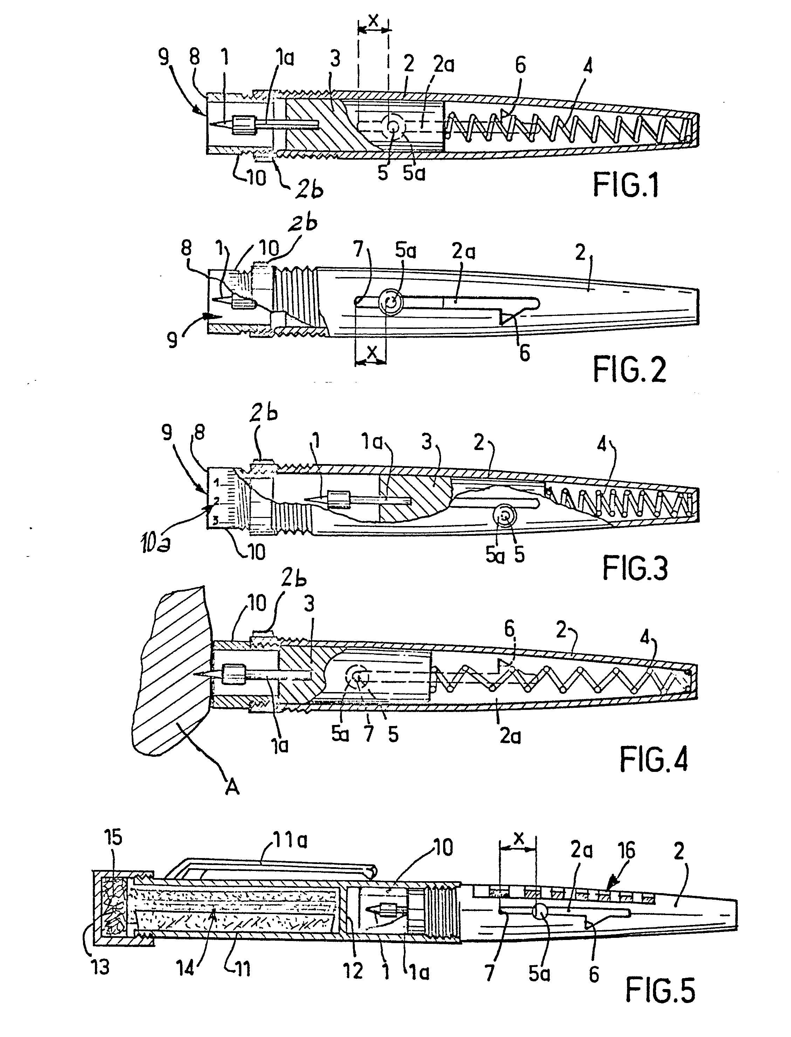

Lorsque le ressort 4 est en position stable de repos, ainsi que cela est représenté sur la figure 1, sa longueur est telle que la pointe 1 est située au plus au niveau du bord 8 de l'orifice 9 par lequel elle doit s'extraire du corps 2 et, avec l'exemple représenté au dessin, la pointe 1 est même nettement en deça de ce bord 8.

Ainsi, lorsque le dispositif n'est pas utilisé, la pointe 1 est encore abritée et ne peut pas accidentellement blesser l'utilisateur.

On voit que dans cette position (figures 1 et 2) la tige 5 est située nettement avant la butée 7 puisqu'elle est séparée de celle-ci d'une distance $x$.

Pour armer le dispositif afin qu'il soit prêt à être utilisé, on agit sur le bouton 5a pour amener la tige 5 en regard de la butée 6, ce qui est obtenu en comprimant le ressort 4 par coulissement de la monture 3 dans le corps 2, la tige 5 passant librement dans la lumière de 2a. Lorsque le bouton 5a arrive au maximum de sa course, on fait légèrement pivoter l'ensemble transversalement à l'axe général du corps 2 pour que la tige 5 se place en regard de la butée 6, ce qui immobilise l'ensemble dans cette position (figure 3).

On place alors le bord 8 de l'orifice 9 contre un endroit A du corps (qui est généralement un doigt de la main) et l'on fait pivoter le bouton 5 dans le sens inverse du précédent pour que la tige 5 quitte la butée 7 et retrouve la lumière 2a. La monture mobile n'est donc plus retenue et le ressort 4 se détend brusquement de sorte qu'il projette la monture 3 en dépassant sa position stable de repos (figures 1 et 2), ce qui a pour effet d'obliger la pointe 1 à dépasser le bord 8 de l'orifice 9 et donc à pénétrer dans l'épiderme A du patient.

La tige 5 atteint finalement la butée 7 qui empêche l'extraction complète de la monture mobile 3 et/ou de la pointe 1 et qui détermine avec précision la course de la pointe 1 hors du dispositif, c'est-à-dire sa pénétration dans l'épiderme du patient. Lorsque le ressort 4 a dépassé sa position stable de repos, il revient à celle-ci en réintégrant la pointe 1 à l'intérieur du corps 2, de sorte que l'ensemble retrouve la position neutre représentée sur les figures 1 et 2 dans laquelle la pointe 1 se retrouve à l'abri. L'extrémité du corps 2 est réalisée dans une matière relativement rigide et il faut que la pointe 1 soit située assez près du bord 8, pour que l'on soit assuré qu'au moment de la brusque détente du ressort 4, la pointe 1 s'extraie sur une distance suffisamment longue pour piquer correctement.

Selon un mode de réalisation de l'invention, et comme cela est représenté au dessin, l'extrémité du corps 2 où se trouve l'orifice 8 par lequel la pointe 1 doit s'extraire, est constituée par un manchon vissé 10 dont l'avancée par rapport au corps 2 est ainsi réglable.

Grâce à cette disposition, la pointe 1 peut pénétrer plus ou moins profondément dans l'épiderme, selon que le manchon 10 est plus ou moins vissé.

Cela revient, en somme, à régler la longueur totale du corps 2.

Grâce à ces dispositions, chaque usager peut régler la profondeur de pénétration souhaitée selon sa constitution ou selon sa préférence.

Pour faciliter ce réglage, on prévoit une graduation 10a sur le manchon 10 et un repère 2b sur le corps 2.

L'arrêt de la tige 5 par la butée 7 détermine sa course maximum de sorte que, finalement, la pénétration est réglable mais constante chez un même sujet.

La longueur du manchon 10 peut être fixée à une valeur telle que la pointe 1 soit facilement accessible quand le manchon 10 est retiré, afin de remplacer la pointe 1 et/ou de procéder aisément à un nettoyage parfait. Naturellement, on peut volontairement provoquer l'extraction lente et contrôlée de la pointe 1 en actionnant le bouton 5a vers la butée 7.

La forme caractéristique d'un dispositif conforme à l'invention est celle d'un stylographe car elle permet non seulement une bonne tenue par la main qui opère mais également une excellente visibilité de l'endroit précis où la piqûre doit être effectuée car aucune partie du corps 2 ne dépasse le diamètre de son extrémité qui peut être relativement faible et se rapprocher de celui d'une pointe de stylomine ou de stylo à bille.

Selon l'invention, le corps 2 en forme de stylographe comprend un capuchon 11 qui est fermé par une cloison transversale 12 pour constituer un réceptable susceptible d'être obturé par un bouchon 13.

Ainsi, on peut placer dans le capuchon 11 une petite réserve de bandelettes 14 de tout type connu en soi et destinées à recevoir par capillarité la goutte de sang apparaissant après piqûre.

Les caractéristiques de capillarité de ces bandelettes ont pour conséquence que celles-ci sont particulièrement sensibles à l'humidité de sorte que, conformément à l'invention, le capuchon 11 peut être muni intérieurement d'un produit absorbant l'humidité et, notamment, une capsule dessiccante 15.

La forme allongée et mince du corps 2 le rend particulièrement apte à présenter une échelle colorimétrique 16 de tout type connu en soi.

Ainsi, l'usager recueille une goutte de sang avec une bandelette 14 puis, lorsque le réactif qui imprègne la bandelette 14 a fait son effet, l'utilisateur approche la bandelette 14 de l'échelle colorimétrique 16 pour observer et reconnaître la partie colorée de l'échelle 16 qui est la plus voisine de la bandelette 14 utilisée pour savoir quel taux de sucre contient son sang.

L'échelle colorimétrique 16 peut, par exemple, être celle qui est généralement placée dans la boîte neuve de bandelettes 14 auquel cas on prévoit sur le corps 2 un petit logement transparent dans lequel on glisse l'échelle colorimétrique.

Celle-ci est donc continuellement protégée et n'est soumise à aucune usure. Elle ne peut pas non plus être perdue car après usage l'utilisateur remet le capuchon 11 sur le corps 2, par exemple au moyen d'un pas de vis comme cela est représenté, et l'ensemble peut être placé dans une poche comme un stylo traditionnel grâce à une bague 11a.

Il doit être bien compris que le dispositif qui vient d'être décrit peut recevoir des modifications, tout particulièrement en ce qui concerne le mécanisme de commande car l'homme de métier sait qu'il existe divers moyens d'obtenir une brusque détente d'un ressort et son maintien en position bandée.

La position relative de la pointe 1 et du bord 8 du corps 2 (ici manchon 10) a trois situations caractéristiques:
- position de repos (figures 1 et 2);
- position armée (figure 3);
- position fugace d'utilisation (figure 4).

L'invention ne porte pas sur les moyens d'assujettissement du manchon 10 et du capuchon 11 sur le corps 2 ou du bouchon 13 sur le capuchon 11 car on peut utiliser pour cela différents moyens bien connus et autres que des pas de vis, ne serait-ce qu'en raison du prix relativement élevé de ces pas de vis lorsque les pièces en cause doivent être obtenues par moulage de matières synthétiques, ce qui est recommandé ici.

REVENDICATIONS

1.- Dispositif pour provoquer une petite piqûre au moyen d'une pointe(1)en vue de recueillir une goutte de sang devant être étudiée, notamment par mise en contact avec un réactif, du type comprenant un corps (2) dans lequel une monture porte-pointe (3) est montée mobile et est sollicitée par un ressort (4) entre une position d'effacement à l'intérieur du corps (2) et une position d'extraction maximum hors du corps (2) déterminée par une butée (7) avec laquelle la monture (3), ou une pièce (5) solidaire de cette dernière, doit coopérer pour déterminer la position d'extraction maximum, au-delà de la position naturelle déterminée par la longueur du ressort (4) au repos, caractérisé en ce que la butée (7) est fixe tandis que la longueur totale du corps (2) est réglable au moyen d'une pièce (10) reliée au corps (2), ajustable en position mais indépendante des pièces mobiles (1-3-4-5).

2.- Dispositif selon la revendication 1, caractérisé en ce que la pièce (10) est constituée par un manchon situé à l'extrémité du corps (2) où se trouve l'orifice (8) par lequel la pointe (1) doit s'extraire.

3.- Dispositif selon la revendication 1, caractérisé en ce que la pièce (10) porte une graduation (10a) située en regard d'un repère (2b) du corps (2).

4.- Dispositif selon la revendication 1, caractérisé en ce que le corps (2) a la forme générale d'un stylographe et en ce qu'il comprend un capuchon (11) fermé par une cloison transversale (12) pour constituer un réceptacle susceptible d'être obturé par un bouchon (13) et devant contenir des bandelettes (14) de tout type connu en soi et destinées à recevoir par capillarité la goutte de sang apparaissant après piqûre, ce réceptacle pouvant être muni intérieurement d'un produit absorbant l'humidité tel qu'une capsule dessiccante (15).

FIG.1

FIG.2

FIG.3

FIG.4

FIG.5

## DOCUMENTS CONSIDERES COMME PERTINENTS

| Categorie | Citation du document avec indication, en cas de besoin, des parties pertinentes | Revendication concernee | CLASSEMENT DE LA DEMANDE (Int. Cl. 3) |
|---|---|---|---|
| X | FR - A - 2 365 331 (A.H. SUTOR & HELLIGE GmbH) <br><br> * page 3; en entier - page 4, ligne 20; figure 1 * <br><br> -- | 1,2 | A 61 B 5/14 <br> 17/32 |
| X | US - A - 3 030 959 (H. GRUNERT) <br><br> * colonne 1, lignes 32-58; colonne 2, ligne 47 - colonne 4, ligne 33; figures 1-4 * <br><br> -- | 1-4 | |
| X | GB - A - 741 774 (H.G. EAST) <br><br> * page 2, colonne de gauche, ligne 17 - colonne de droite, ligne 83; figure 1 * <br><br> -- | 1,2 | |
| Y | DE - C - 867 424 (A. BECK) <br><br> * en entier * <br><br> -- | 1,2 | A 61 B <br> A 61 M |
| D,Y | DE - C - 459 483 (E. HENCKE) <br><br> * en entier * <br><br> -- | 1 | |
| A | DE - C - 866 829 (T. PETERS) <br><br> * en entier * ./. <br><br> -- | 1 | |

**DOMAINES TECHNIQUES RECHERCHES (Int. Cl. 3)**

Le présent rapport de recherche a été établi pour toutes les revendications

| Lieu de la recherche | Date d'achevement de la recherche | Examinateur |
|---|---|---|
| La Haye | 15-09-1982 | ZILLIOX |

Office européen

des brevets

## REVENDICATIONS DONNANT LIEU AU PAIEMENT DE TAXES

La présente demande de brevet européen comportait lors de son dépôt plus de dix revendications.

☐ Toutes les taxes de revendication ayant été acquittées dans les délais prescrits, le présent rapport de recherche européenne a été établi pour toutes les revendications.

☐ Une partie seulement des taxes de revendication ayant été acquittée dans les délais prescrits, le présent rapport de recherche européenne a été établi pour les dix premières revendications ainsi que pour celles pour lesquelles les taxes de revendication ont été acquittées.

à savoir les revendications:

☐ Aucune taxe de revendication n'ayant été acquittée dans les délais prescrits, le présent rapport de recherche européenne a été établi pour les dix premières revendications.

## X | ABSENCE D'UNITE D'INVENTION

La division de la recherche estime que la présente demande de brevet européen ne satisfait pas à l'exigence relative à l'unité d'invention et concerne plusieurs inventions ou pluralités d'inventions.

à savoir:

1. revendications 1-3
2. revendication 4

☒ Toutes les nouvelles taxes de recherche ayant été acquittées dans les délais impartis, le présent rapport de recherche européenne a été établi pour toutes les revendications.

☐ Une partie seulement des nouvelles taxes de recherche ayant été acquittée dans les délais impartis, le présent rapport de recherche européenne a été établi pour les parties de la demande de brevet européen qui se rapportent aux inventions pour lesquelles les taxes de recherche ont été acquittées

à savoir les revendications:

☐ Aucune nouvelle taxe de recherche n'ayant été acquittée dans les délais impartis, le présent rapport de recherche européenne a été établi pour les parties de la demande de brevet européen qui se rapportent à l'invention mentionnee en premier lieu dans les revendications.

à savoir les revendications:

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| A | <u>FR - A - 2 100 407</u> (F. GORBAHN)<br><br>* page 1, ligne 12 — page 2, ligne 20; page 6, lignes 2-14; figures 1-18 * | 4 | |
| | ------- | | **DOMAINES TECHNIQUES RECHERCHES (Int. Cl.³)** |